# EUROPÄISCHE PATENTSCHRIFT

(11) **EP 0 826 336 B1**
(45) Veröffentlichungstag und Bekanntmachung des Hinweises auf die Patenterteilung: **19.09.2001**
(21) Anmeldenummer: 97112686.7
(22) Anmeldetag: 24.07.1997
(51) Int. Cl.: A61B 5/22, A61B 5/20

(54) **Verwendung einer Vorrichtung zur Erfassung der Innervation der Beckenbodenmuskulatur, der analen Schliessmuskulatur oder der Pubococcygeusmuskulatur**
Use of a device for detecting the innervation of the pelvic floor and of anal sphincters
Utilisation d'un dispositif pour détecter l'innervation du plancher pelvien et du sphincter anal

(30) Priorität: 28.08.1996 DE 29614895 U
(43) Veröffentlichungstag der Anmeldung: 04.03.1998
(73) Patentinhaber: Schönfeld, Andreas, Dipl.-Ing., 76189 Karlsruhe (DE)
(72) Erfinder: Schönfeld, Andreas, Dipl.-Ing., 76189 Karlsruhe (DE)
(74) Vertreter: Dimmerling, Heinz, Dipl.-Ing.

(56) Entgegenhaltungen:
- CH-A- 591 234
- DE-A- 3 619 695
- DE-A- 4 038 853
- US-A- 5 411 548

## Beschreibung

Die Erfindung betrifft die Verwendung einer Vorrichtung zur Bestimmung der Innervation der Beckenbodenmuskulatur, der analen Schließmuskulatur oder der Pubococcygeusmuskulatur, mit einem Sensor, einer Signalaufbereitungseinheit, einer Anordnung zur Erfassung des Signalspitzenwertes, und einer Signalanzeige.

Zur Behebung der Inkontinenz bei Menschen sind Geräte bekannt, mit denen die Aktivität der Beckenbodenmuskulatur, der analen Schließmuskulatur oder der Pubococcygeusmuskulatur erfaßt werden kann. Die Aktivität der Muskulatur wird mittels eines Elektromyogramms (EMG) erfaßt. Die Kraftentfaltung der betreffenden Muskulatur wird über eine Anzeige, welche beispielsweise aus hintereinander angeordneten Leuchtdioden bestehen kann, ausgegeben. Durch die Rückkopplung, die die betreffende Person durch die angezeigte Aktivität beziehungsweise Kontraktionskraft der betreffenden Muskulatur erhält, kann die Person motiviert werden, die Intensität der Muskelaktivität zu steigern. Trainingsziel der das Training absolvierenden Person ist es, eine möglichst hohe dauerhafte Anspannung der Muskeln zu erreichen. Durch eine Steigerung der betreffenden Muskelaktivität kann die Inkontinenz stark reduziert und letztendlich sogar behoben werden.

Aus der DE 94 16 826 U ist eine Vorrichtung zum Trainieren der Schließmuskeln bekannt, bei der in den Analkanal ein Sensor eingeführt wird, welcher die bei der Betätigung der Schließmuskeln entstehenden elektrischen Potentialen erfaßt und an eine elektronische Auswerteeinheit weiterleitet. Das Sensorsignal wird zunächst in einer Signalverarbeitungseinheit aufbereitet und an eine Anzeigensteuerung geleitet. Mittels der Anzeigensteuerung werden Leuchtdioden angesteuert. Das Sensorsignal wird in der Signalverarbeitungseinheit im wesentlichen gefiltert. Um eine flakkerfreie Anzeige des Signals zu erreichen, wird das Signal über einen Tiefpaß geführt. Hierdurch werden störende Signalspitzen ausgeglichen, wodurch eine ruhige Anzeige erreicht wird.

Aus der DE 40 38 853 A1 ist eine Vorrichtung zur Funktionsdiagnose des Kontinenzorgans bekannt, welche einen Sensor, eine Signalaufbereitungseinheit, eine Anordnung zur Erfassung mehrerer Meßwerte und eine Signalanzeige aufweist. Mittels der bekannten Vorrichtung kann eine Inkontinenztherapie und deren therapeutischer Erfolg überwacht werden.

Der CH 591234 A läßt sich ein Gerät zur Ermittlung der maximalen Kontraktionskraft und der maximalen Kontraktions- und Erschlaffungsgeschwindigkeit eines isoliert kontrahierenden Körperorgans entnehmen, bei welchem der von einem Sensor abgegebene Spitzenwert erfaßt wird.

Darüber hinaus ist aus der US-A-5,411,548 eine Vorrichtung zur Erfassung eines Elektromyogramms bekannt, welche einen Sensor, eine Signalaufbereitungseinheit und eine Signalanzeige aufweist. Aus der Druckschrift ist auch bekannt, den Signalspitzenwert zu erfassen, um daraus auf den Wert der höchsten Muskelkontraktion zu schließen.

Es wurde nun herausgefunden, daß mittels derartiger Trainingsgeräte neben der Stuhlinkontinenz besonders eine bestimmte Form der Harninkontinenz, die sogenannte Streßinkontinenz verbesserbar beziehungsweise behebbar ist. Des weiteren wurde herausgefunden, daß verbesserbare beziehungsweise behebbare Streßinkontinenz nur dann vorliegt, wenn sich die Innervation der Beckenbodenmuskulatur, der analen Schließmuskulatur oder der Pubococcygeusmuskulatur in einem bestimmten Bereich befindet. Hinweise darauf, eine, Feststellung zu treffen, ob sich die Innervation dieser Muskulatur in einem bestimmten Bereich befindet, geben die bekannten Geräte nicht. Der Innervationsgrad gibt an, wie stark beispielsweise die Beckenbodenmuskulatur an das Nervensystem angebunden ist.

Gemäß der Erfindung ist vorgesehen, eine eingangs genannte Anordnung zur Bestimmung zu Verwenden, ob eine durch Training verbesserbare Form der Inkontinenz vorliegt. Mittels der Anordnung zur Erfassung des Signalspitzenwertes ist es möglich, einen nur kurzzeitig auftretenden Spitzenwert der Muskelaktivität zu erfassen, welcher dann unabhängig von der eigentlichen Kraft und Ausdauer der Muskulatur den Grad der nervösen Erregung angibt. Durch den Spitzenwert ist es möglich, eine Aussage darüber zu treffen, ob die Muskulatur noch trainierbar, d.h. ob eine Verbesserung der Inkontinenz mittels der bekannten Trainingsgeräte erreicht werden kann.

Liegt der Spitzenwert unterhalb eines ersten bestimmten Wertes, ist die Muskulatur bereits so geschwächt, daß sie mittels der mit den bekannten Trainingsgeräten durchführbaren physischen Trainingsmethode nicht mehr aufgebaut werden kann. Die Muskulatur muß dann zunächst mittels anderer, beispielsweise auch pharmazeutischer Mittel in einen Zustand gebracht werden, in dem sie wieder mittels der bekannten Trainingsgeräte trainierbar ist.

Übersteigt der Spitzenwert einen zweiten bestimmten Wert, ist die Muskulatur so stark innerviert, daß ein Trainieren der Muskulatur mittels der bekannten Trainingsgeräte keine Verbesserung der Inkontinenz bringen würde. Die Inkontinenz muß dann ebenfalls mittels anderer Methoden, gegebenenfalls mit pharmazeutischen Mitteln behandelt werden.

Befindet sich der Spitzenwert jedoch oberhalb des ersten und unterhalb des zweiten bestimmten Wertes, bedeutet dies, daß Streßinkontinenz vorliegt, welche durch ein Trainieren der Muskulatur mittels der bekannten Trainingsgeräte verbesserbar oder behebbar ist. Der Therapeut ist mittels der erfindungsgemäßen Vorrichtung in der Lage festzustellen, ob eine durch Muskeltraining verbesserbare Streßinkontinenz vorliegt.

In besonders vorteilhafter Weise ist in erfindungsgemäßer Weise ein Speicher vorgesehen, welcher den Signalspitzenwert speichert. Hierdurch läßt sich ohne hohe Aufmerksamkeit feststellen, welchen Spitzenwert das Sensorsignal und damit die Muskelaktivität beziehungsweise die Kontraktionskraft der Muskulatur erreicht hat. Das in dem Speicher gespeicherte Signal kann mittels einer digitalen Anzeige ausgegeben werden oder in Form einer analogen Anzeige wie beispielsweise eine Leuchtdiodenreihe dargestellt werden. Bei einer weiteren besonderen Ausführungsform der Erfindung ist vorgesehen, daß der Signalspitzenwert mittels eines Komparators mit wenigstens einem vorbestimmtem Wert verglichen wird. In vorteilhafter Weise wird der Signalspitzenwert jedoch mit zwei vorbestimmten Werten verglichen, wobei der untere Wert die untere Schwelle und der obere Wert die obere Schwelle des Bereichs darstellt, in dem Inkontinenz mittels eines Muskeltrainings verbesserbar ist. Zur Anpassung der Vorrichtung an unterschiedliche Patienten sind die Schwellwerte einstellbar. Das Ausgangssignal des Komparators kann dann dazu verwendet werden anzuzeigen, ob die Muskulatur zu schwach ist, sich in einem trainierbaren Bereich befindet oder so stark ist, daß ein Training der Muskulatur keine Verbesserung der Inkontinenz bringen würde.

Bei einer weiteren vorteilhaften Ausführungsform der Erfindung ist eine Steuerung vorgesehen, mittels der die Anordnung aktivierbar ist. Durch die Steuerung kann die Zeit festgelegt werden, in der die Spitzenwertermittlung stattfinden soll. Der Beginn und das Ende der Meßzeit kann durch manuelle Betätigung von Schaltern festgelegt werden oder automatisch erfolgen. Für automatischen Betrieb kann beispielsweise die Steuerung so ausgebildet sein, daß sie in bestimmten Zeitabständen einen eine bestimmte Zeit andauernden Meßvorgang auslöst, oder daß der Meßvorgang mittels eines Schalters manuell eingeleitet und mittels der Steuerung nach einer bestimmten Zeit automatisch beendet wird.

Weitere Einzelheiten, Merkmale und Vorteile der vorliegenden Erfindung ergeben sich aus der nachfolgenden Beschreibung besonderer Ausführungsbeispiele unter Bezugnahme auf die Zeichnung.

Es zeigt
- Figur 1:: Eine erste Ausführungsform der erfindungsgemäßen Vorrichtung und
- Figur 2:: Eine zweite Ausführungsform der erfindungsgemäßen Vorrichtung.

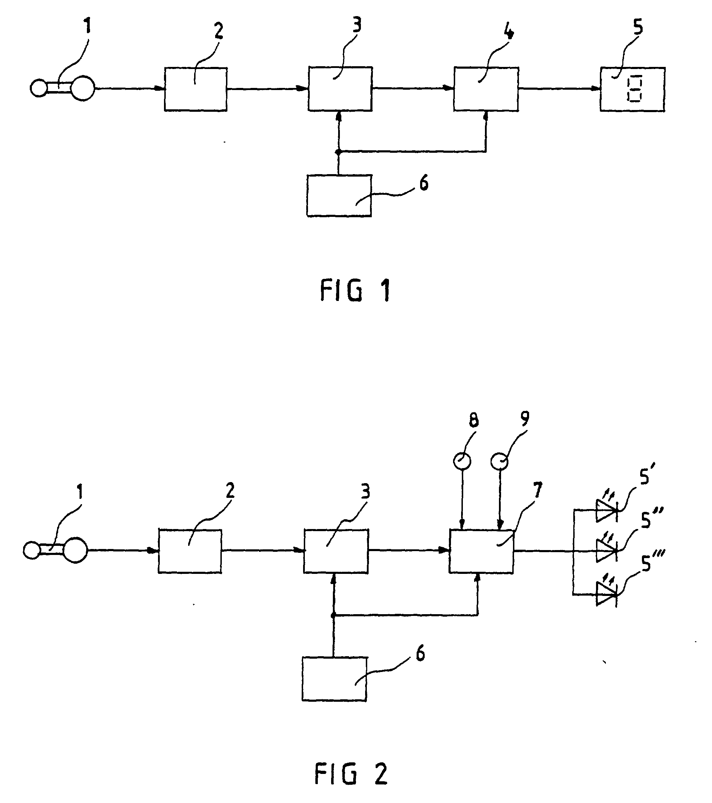

In der Zeichnung sind gleiche Elemente mit gleichem Bezugszeichen versehen. Wie der Figur 1 entnommen werden kann, ist ein Sensor 1, welcher in den Analkanal oder in die Vagina eingeführt wird, mit einer Signalaufbereitungseinheit 2 verbunden. In der Signalaufbereitungseinheit 2 werden aus dem vom Sensor 1 abgegebenen Gesamtsignal spezifische Signalanteile extrahiert. Das von der Signalaufbereitungseinheit 2 abgegebene Signal wird auf eine Anordnung 3 zur Erfassung des Signalspitzenwertes gegeben. Die Anordnung 3 ist mit einer Steuerung 6 verbunden, mittels welcher sie aktivierbar ist. Durch die Steuerung 6 wird somit festgelegt, über welchen Zeitraum die Anordnung 3 den Spitzenwert des von der Signalaufbereitungseinheit 2 abgegebenen Signals erfaßt. Die Anordnung 3 ist des weiteren mit dem Eingang eines Speichers 4 verbunden, welcher den von der Anordnung 3 erfaßten Signalspitzenwert speichert. Das im Speicher 4 gespeicherte Signal wird auf eine Anzeige 5 gegeben, welche das Signal in numerischer Form darstellt. Der Speicher 4 ist auch mit der Steuerung 6 verbunden, welche den im Speicher 4 gespeicherten Wert jeweils zu Beginn eines neuen Meßzyklus zurücksetzt.

Wie der Figur 2 zu entnehmen ist, kann statt des Speichers 4 ein Komparator 7 vorgesehen sein, welcher den von der Anordnung 3 erfaßten Signalspitzenwert mit zwei Schwellwerten 8, 9 vergleicht. Das Ausgangssignal des Komparators 7 wird auf drei Leuchtdioden 5', 5",5"' gegeben. Ist der Signalspitzenwert unterhalb des ersten vorbestimmten Wertes 8, leuchtet die Diode 5'. Ist der Signalspitzenwert größer als der zweite vorbestimmte Wert 9, leuchtet die Diode 5"'. Befindet sich der Signalspitzenwert zwischen den zwei Schwellwerten 8, 9, leuchtet die Diode 5". Der Komparator 7 ist mit der Steuerung 6 verbunden, welche das Ausgangssignal des Komparators 7 zu Beginn jedes neuen Meßzyklus zurücksetzt.

## Patentansprüche

1. Verwendung einer Vorrichtung zur Erfassung des Inervationsgrades der Beckenbodenmuskulatur, der analen Schließmuskulatur oder der Pubococcygeusmuskulatur, mit einem Sensor (1), einer Signalaufbereitungseinheit (2) einer Anordnung (3) zur Erfassung des Signalspitzenwertes und einer Signalanzeige (4), zur Bestimmung, ob eine durch Training verbesserbare Form der In kontinenz vorliegt.

2. Verwendung einer Vorrichtung nach Anspruch 1,
**dadurch gekennzeichnet,**
**daß** ein Speicher (4) vorgesehen ist, zur Speicherung des Signalspitzenwertes.

3. Verwendung einer Vorrichtung nach Anspruch 1,
**dadurch gekennzeichnet,**
**daß** ein Komparator (7) vorgesehen ist, welcher den Signalspitzenwert mit wenigstens einem vorbestimmten Wert (8, 9) vergleicht.

4. Verwendung einer Vorrichtung nach einem der Ansprüche 1 bis 3,
**dadurch gekennzeichnet,**
**daß** eine Steuerung (6) vorgesehen ist, mittels der die Anordnung (3) aktivierbar ist.

## Claims

1. Use of an apparatus for detecting the degree of innervation of the pelvic floor musculature, the anal sphincter musculature or the pubococcygeus musculature, comprising a sensor (1), a signal processing unit (2), an arrangement (3) for detecting the signal peak value, and a signal display (4), for determining whether a form of incontinence which can be improved by training is present.

2. Use of an apparatus according to claim 1 **characterised in that** there is provided a memory (4) for storage of the signal peak value.

3. Use of an apparatus according to claim 1 **characterised in that** there is provided a comparator (7) which compares the signal peak value to at least one predetermined value (8, 9).

4. Use of an apparatus according to one of claims 1 to 3 **characterised in that** there is provided a control (6) by means of which the arrangement (3) can be activated.

## Revendications

1. Utilisation d'un dispositif pour détecter le degré d'innervation de la musculature du plancher pelvien, de la musculature du sphincter anal ou de la musculature de la région pubo-coccygienne, avec un détecteur (1), une unité de traitement du signal (2), une installation (3) pour détecter la valeur de crête du signal et un indicateur de signal (4), afin de déterminer s'il se présente une forme d'incontinence pouvant être améliorée par rééducation.

2. Utilisation d'un dispositif selon la revendication 1, **caractérisée en ce qu'**il est prévu une mémoire (4), pour l'enregistrement de la valeur de crête du signal.

3. Utilisation d'un dispositif selon la revendication 1, **caractérisée en ce qu'**il est prévu un comparateur (7), qui compare la valeur de crête du signal à au moins une valeur prédéterminée (8, 9).

4. Utilisation d'un dispositif selon l'une des revendications 1 à 3, **caractérisée en ce qu'**il est prévu un dispositif de commande (6), qui permet d'activer l'installation (3).
